# EUROPEAN PATENT APPLICATION

(11) **EP 2 853 191 A2**
(43) Date of publication of application: **01.04.2015**
(21) Application number: 14186756.4
(22) Date of filing: 29.09.2014
(51) Int. Cl.: A61B 1/04, G09G 5/02, H04N 17/02

(54) **Image processing apparatus, image processing system, and image processing method**

(30) Priority: 30.09.2013 JP 2013204375
(71) Applicant: Fujifilm Corporation, Minato-ku Tokyo (JP)
(72) Inventor: Miura, Nobuyuki, Tokyo (JP)
(74) Representative: Klunker . Schmitt-Nilson . Hirsch

(57) **Abstract**

An image processing apparatus includes a reference image signal outputting unit (27) that outputs a reference image signal in which color information of a plurality of colors is included in a chart image in a display plane or a time axis, a color display information detection unit that detects color display information of an image displayed on a displaying unit (25) based on the reference image signal, a profile generation unit (31) that generates a display characteristic profile of the displaying unit using the detected color display information and the color information of the chart image, a display characteristic correcting unit (35) that generates a color correction image signal obtained by correcting an image signal to be output which is input from a modality based on the display characteristic profile, and a standardization processing unit (37) that converts the generated color correction image signal to a standardization image signal.

## Description

### BACKGROUND

### 1. Technical Field

The present invention relates to an image processing apparatus, an image processing system, and an image processing method.

### 2. Related Art

A system which stores an observation image data obtained by capturing an image by various modalities such as an endoscope device and allows the stored observation image data to be shared through a network to which the modalities are connected is included in a PACS (Picture Archiving and Communication Systems). According to the system, an observation image data file stored in the modality side may be extracted to be displayed by an external image display apparatus connected to the network.

Further, in recent years, standardization of a communication protocol for a medical image data which enables interconnection between medical image instruments such as image diagnosis apparatuses or medical information servers manufactured by different makers is being progressed. In such a standard, contents or a data structure of the medical information such as image information or patient information, or a sequence or an interface when communicating the medical information is defined in detail, and a color space which becomes a reference of color information of an image signal is also discussed as one of the standards.

The observation image data described above is required to display a display image of the observation image data with a color which is always the same as that at the time of examining the display image by the modality even when the display image of the observation image data is displayed in a certain external image display apparatus connected to the network. In general, several technologies for displaying the display image with the same color in a plurality of display apparatuses have been suggested, and may include technologies disclosed in, for example, Patent Literature 1 (JP-A-H6-208360) and Patent Literature 2 (JP-A-2010-212937).

### SUMMARY OF INVENTION

In the technology disclosed in Patent Literature 1, when an image which is the same as that displayed in a standard display apparatus is displayed in a specific display apparatus, information about a color display characteristic of the standard display apparatus is registered in a calibration table in advance and an image signal to be output to the specific display apparatus is corrected using the calibration table. However, although the image signal is corrected according to a display characteristic of the standard display apparatus, a color may not be correctly displayed due to a difference of the standard when the specific display apparatus is a display system having a standard different from that of a reference display apparatus. The technology disclosed in Patent Literature 2 is that display contents of the display apparatus are actually measured to calibrate chromaticity but is that a target chromaticity is adjusted to an average level in a display screen so as to change the target chromaticity of a reference display apparatus itself according to the display characteristic of other display apparatus. Further, a standardization image data based on the standardized standard may be used in order to display the image with the same color in a plurality of display apparatuses, but the endoscope device or the external image display apparatus may not be an instrument which does not compatible with the standardization image data due to the difference of makers or settings of the display functions.

In an endoscopic diagnosis, the condition of a patient may be judged from subtle color difference of the observation image data and a high quality color reproduction is required especially for displaying the observation image data. Further, the observation image data by the endoscopic diagnosis may be frequently displayed in an image display apparatus other than the endoscope device which has conducted the endoscopic examination in such a case where, for example, an examined image is attached to an endoscopic examination result report or a pathological image is attached to an examination report of pathological examination division, including a case where the observation image data is redisplayed in order to refer to the observation image after the endoscopic examination is completed,

Accordingly, the present invention intends to provide an image processing apparatus, an image processing system and an image processing method which may display an output image signal in the same color and enable sharing of the correct image information even when an output image signal to be output to a displaying unit of a modality is displayed in any external image display apparatus, regardless of difference in instrument makers or settings of display functions.

The present invention relates to the following aspects.
(1) According to an aspect of the present invention, it provides an image processing apparatus which receives from a modality, an image signal to be output to a displaying unit of the modality and outputs a standardization image signal obtained by standardizing the image signal to be output, the image processing apparatus including: a reference image signal outputting unit that outputs a reference image signal in which color information of a plurality of colors is included in a chart image in a display plane or a time axis; a color display information detection unit that detects color display information of an image displayed on the displaying unit based on the reference image signal; a profile generation unit that generates a display characteristic profile of the displaying unit using the detected color display information and the color information of the chart image; a display characteristic correcting unit that generates a color correction image signal obtained by correcting the image signal to be output which is input from the modality based on the display characteristic profile; and a standardization processing unit that converts the generated color correction image signal to the standardization image signal.
(2) According to another aspect of the present invention, it provides an image processing system, including the image processing apparatus; the modality provided with the displaying unit; and an external image display apparatus that displays the standardization image signal.
(3) According to another aspect of the present invention, it provides an image processing method for receiving from a modality, an image signal to be output to a displaying unit of the modality and outputting a standardization image signal obtained by standardizing the image signal to be output, the image processing method including: outputting a reference image signal in which color information of a plurality of colors is included in a chart image in a display plane or a time axis; detecting color display information of an image displayed on the displaying unit based on the reference image signal; generating a display characteristic profile of the displaying unit using the detected color display information and the color information of the chart image; generating a color correction image signal obtained by correcting the image signal to be output which is input from the modality based on the display characteristic profile; and converting the generated color correction image signal to the standardization image signal.

According to any aspect of the present invention, regardless of the difference in instrument makers or settings of display functions, it becomes possible to display an output image signal in the same color and share the correct image even when an output image signal to be output to a displaying unit of a modality is displayed in any external image display apparatus.

### BRIEF DESCRIPTION OF DRAWINGS

FIG. 1 is a block diagram illustrating an image processing system for explaining an embodiment of the present invention.
FIG. 2 is an outer appearance view illustrating an example of an endoscope device and the image processing apparatus.
FIG. 3 is a flowchart illustrating a processing sequence of matching colors of a display image.
FIG. 4 is an explanatory view conceptually illustrating a calibration process by calibration.
FIG. 5 is an explanatory view illustrating a situation where an output image signal to be output to a monitor is input to a capture device from the endoscope device, the capture device generates a standardization image and outputs the generated standardization image to respective external image display apparatuses.

### DESCRIPTION OF EMBODIMENTS

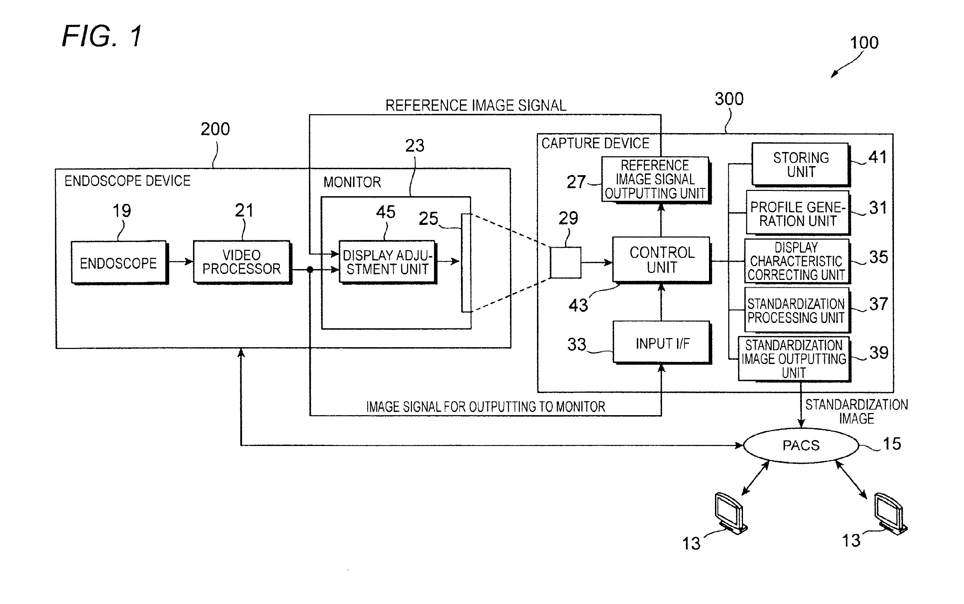

Hereinafter, descriptions will be made on an embodiment of the present invention with reference to the drawings. FIG. 1 is a block diagram illustrating an image processing system for explaining an embodiment of the present invention. An image processing system 100 according to a configuration of the embodiment is provided with an endoscope device 200 which is one of modalities and a capture device 300 as an image processing apparatus, and is connected to a network 15 of the PACS (Picture Archiving and Communication Systems) to which an external image display apparatus 13 is connected.

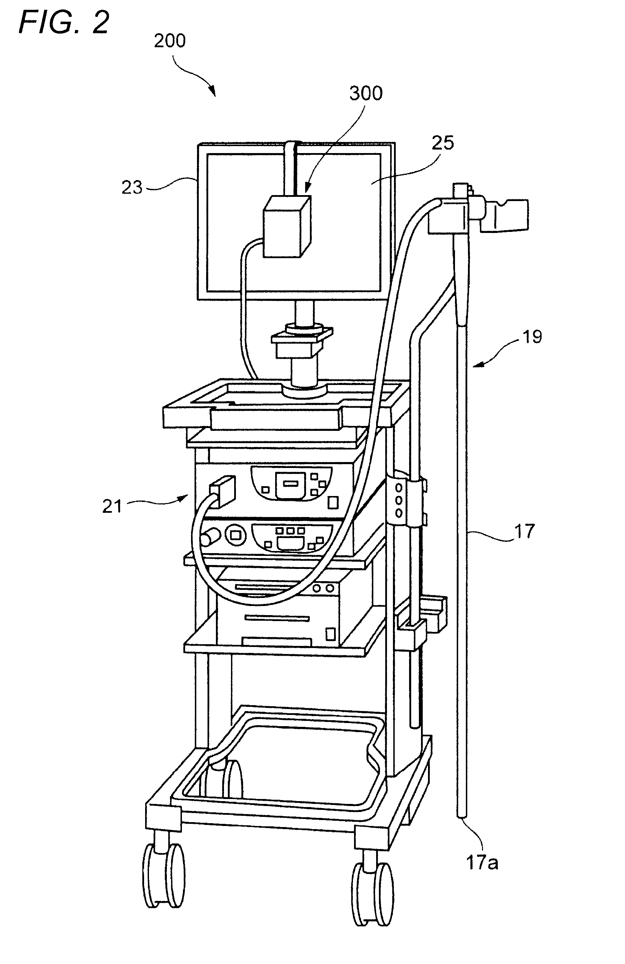

In FIG. 2, an outer appearance view of an example of an endoscope device 200 and the image processing apparatus 300 is illustrated. The endoscope device 200 mainly includes an endoscope 19 provided with an image-capturing unit formed at a front end portion 17a of an endoscope insertion unit having a long shape, a video processor 21 to which an image signal of an observation image captured by the endoscope 19 is input and a monitor 23 as an image displaying unit which is connected to the video processor 21 and displays image information.

The capture device 300 has a calibration function which performs color matching with a display color of the monitor 23. When a calibration mode is set, the capture device 300 displays a chart image in which a plurality of reference colors are contained on the monitor 23 of the endoscope device 200 to detect a display color of the displayed chart image.

Also, the capture device 300 associates the detected display color with the reference color of the chart image to generate a display characteristic profile which represents a display characteristic of the monitor 23. The capture device 300 is configured to be detachable in such a manner that the capture device 300 is disposed to face a displaying unit 25 of the monitor 23 during calibration and is removed from the displaying unit 25 of the monitor 23 after completion of calibration.

As illustrated in FIG. 1, the capture device 300 includes a reference image signal outputting unit 27 which outputs a reference image signal used for displaying the chart image on the monitor 23, an image sensor 29 as a color display information detection unit which captures an image of the chart image displayed on the displaying unit 25 of the monitor 23 to detect the display color of the chart image, and a profile generation unit 31 which generates the display characteristic profile described above.

Further, the capture device 300 includes an I/F 33 to which an image signal which is the same as an image signal to be output to the monitor 23 (image signal to be output) is input to the monitor 23 from the video processor 21, a display characteristic correcting unit 35 of which details will be described later, a standardization processing unit 37, a standardization image outputting unit 39, a storing unit 41 and a control unit 43 such as a central processing unit (CPU) which controls each component.

Further, the monitor 23 includes a display adjustment unit 45 which adjusts, for example, contrast or gain from a standard state in an image display of image signal to be output to the monitor from the video processor 21.

When image information of image signal to be output and displayed on the monitor 23 is displayed on the external image display apparatus 13 by the endoscope device 200, the image processing system 100 having a function which makes the color of the image displayed on the monitor 23 to coincide with that displayed on the external image display apparatus 13 with a high precision.

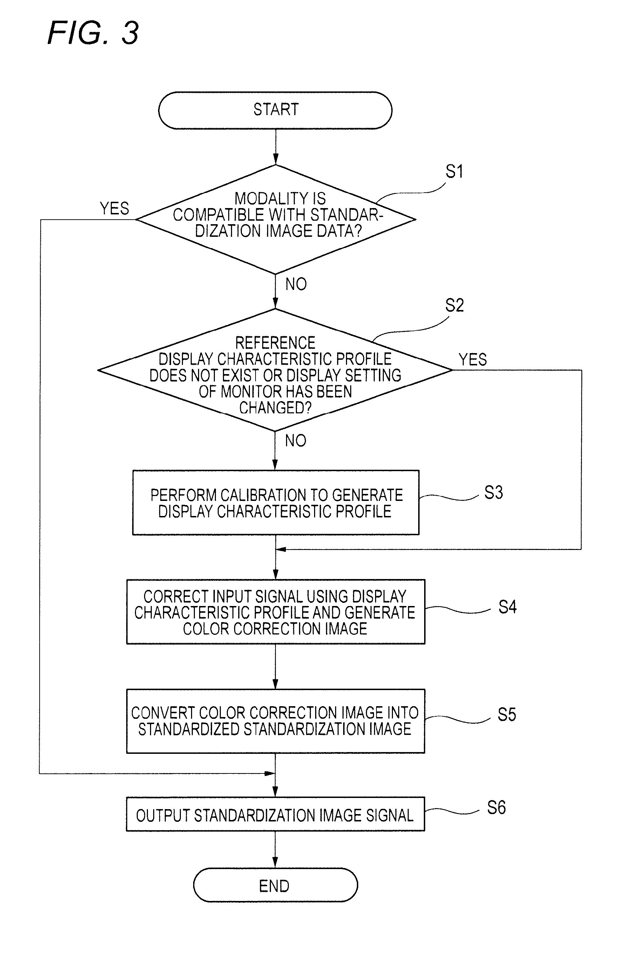

Next, description will be made on a sequence which makes the color of the display image in the monitor 23 to coincide with that in the external image display apparatus 13 by the image processing system 100. In FIG. 3, a flowchart illustrating a processing sequence of matching colors of a display image is illustrated.

First, when the endoscope device 200 is a modality compatible with a standardized standardization image data, the endoscope device 200 outputs a standardization image signal of an image signal to be output to the monitor without the capture device 300 (step S6). The image data of the output standardization image signal is stored in, though not illustrated, a storing unit of the endoscope device 200 or a storage device such as a medical image information server connected to the network 15.

Also, the external image display apparatus 13 reads the image data file stored in the endoscope device 200 according to the standard thereof to display an image. In such a case, the display image displayed on the external image display apparatus 13 by the standardization image data becomes to have the same color as that displayed on the monitor 23 of the endoscope device 200.

However, when the endoscope device 200 is a modality which is not compatible with the standardization image signal described above, the endoscope device 200 is not able to generate the standardization image signal. Therefore, the capture device 300 generates and stores the standardization image signal in the storing unit 41 of the capture device 300 or an external storage device (illustration is omitted) connected to the network 15 as the image data. When the external image display apparatus 13 reads the stored image data to display the image, the image may be displayed with the same color as that of the image displayed on the monitor 23 of the endoscope device 200.

Next, a case where the standardization image signal is generated by the capture device 300 will be described in detail. An instrument installer performs color adjustment of the endoscope device and the monitor according to a sequence prescribed in the endoscope device or instruction by a physician who conducts an endoscopic treatment. It is confirmed whether a standard display characteristic profile for the monitor 23 of the endoscope device 200 does not exist in, such as the storing unit 41, or the display adjustment unit 45 of the monitor 23 has been changed from a standard setting (step S2). When at least one of the conditions is satisfied, calibration is performed to generate the display characteristic profile by an actual measurement (step S3). Further, even in a case other than installation, the calibration is performed according to changes of the display characteristic due to change with time or change of settings of the endoscope device or the monitor.

Specifically, the control unit 43 switches a mode of the capture device 300 to a calibration mode. Also, according to an instruction by the control unit 43, a reference image signal of the chart image on which a plurality of reference color patches or gray scales are arranged in the same plane is output to the monitor 23 from the reference image signal outputting unit 27. The reference image signal may be a reference image signal that the patches or gray scales are displayed in chronological order.

The monitor 23 displays the input reference image signal on the displaying unit 25. The capture device 300 captures the chart image displayed on the displaying unit 25 by the image sensor 29 and obtains chromaticity characteristics of RGB by detecting the display color of the chart image.

In the calibration by using the chart image, color signals of, for example, 16, 770, 000 colors ranging from RGB (0.0.0) to RGB (255, 255, 255) are displayed on the monitor 23 and mapping of all the color signals or a combination of some of the color signals with absolute color space is performed. The chart image may be a chart image which is developed in a coordinate axis on a display plane as well as a chart image which is varied on a time axis.

The control unit 43 outputs information of, such as the display color of the detected chart image or the reference color of the chart image to the profile generation unit 31. The profile generation unit 31 generates the display characteristic profile in which the display color information about the display color of the chart image is associated with the color information about the reference color of the chart image. The display characteristic profile is a LUT (look-up table) in which a driving signal level of the monitor is associated with absolute color space and is used for calibrating respective parameters of various display characteristics such as gradation characteristic, chromaticity or color temperature obtained by the chart image with the prescribed standard characteristic.

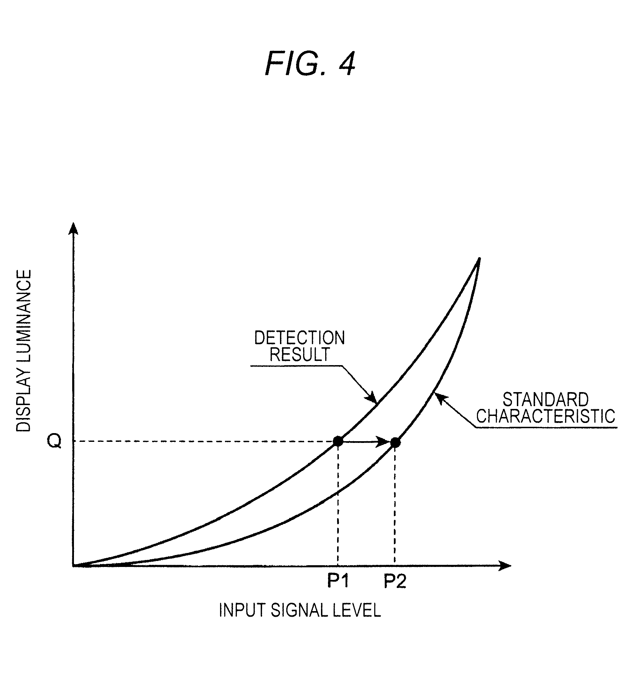

FIG. 4 is an explanatory view conceptually illustrating a calibration process by calibration. The image information which becomes the standardization image is stored in a storing destination connected to the network 15 of the PACS and a RGB luminance level of each pixel of the image is adjusted to a level defined in the LUT. The LUT is changed so that an image to be displayed on the external display apparatus 13 may matched the color displayed on the monitor 23.

In the present calibration, the driving signal level is changed so that the display luminance Q becomes a P2 level of the standard characteristic, when, for example, the input signal level is P1 and the display luminance of the detected result is Q. In actually, two types of LUTs, that is, a LUT which associates the driving signal level with absolute color space in the display characteristic correcting unit 35 and the other LUT which associates the driving signal level with the absolute color space and the standardization color space in the standardization processing unit 37 are used and the former is calibrated by the calibration.

The generated display characteristic profile is stored in the storing unit 41 of the capture device 300 or in the external storage device connected to the network 15 and is provided for correcting the image signal to be output to the monitor.

Next, the control unit 43 receives the image signal to be output to the monitor input from the input I/F 33 as a continuous still image. The display characteristic correcting unit 35 performs the color correction on each of received still images according to the display characteristic of the monitor 23 of the endoscope device 200 using the display characteristic profile, and generates the color correction image (step S4). Alternatively, the color correction may be performed on the still image extracted by the instruction to capture the still image. The color correction is used for reducing a processing load in a case where capturing of the moving image is not needed.

The standardization processing unit 37 converts a color correction image which is undergone the color correction to a predetermined standardized standardization image (step S5). The standardized standardization image, such as sRGB standardization image or adobe RGB standardization image, refers to a standardization image which may be converted with a specific color space. The standardized standardization image is further converted into a DICOM standard format when being transmitted to a DICOM standard PACS. The control unit 43 outputs the converted standardization image from the standardization image outputting unit 39 to the network 15 (step S6).

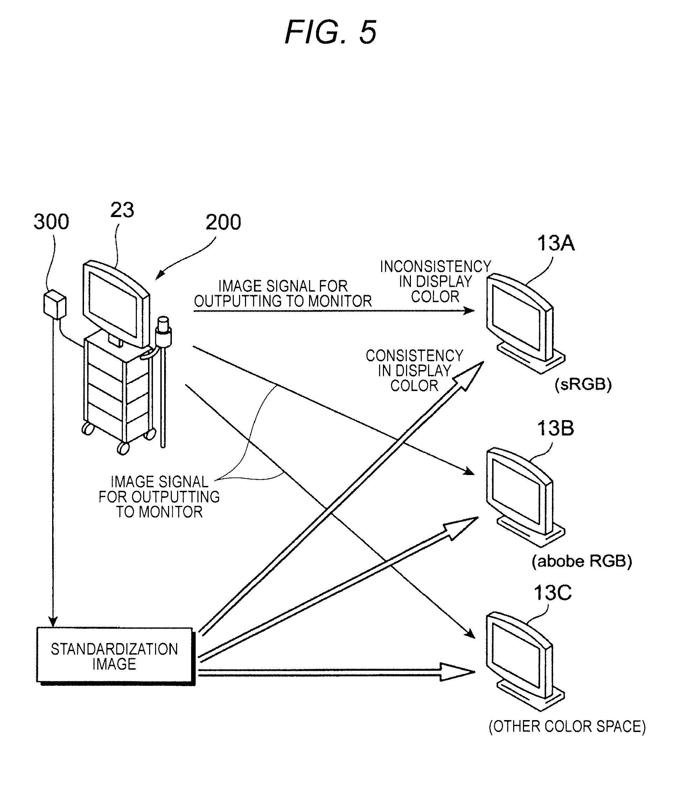

Next, an effect by the image processing system 100 will be described. FIG. 5 is an explanatory view illustrating a situation where an output image signal to be output to a monitor is input to a capture device 300 from the endoscope device, the capture device 300 generates a standardization image and outputs the generated standardization image to respective external image display apparatuses 13A, 13B and 13C.

The external image display apparatus 13A displays the color space image data of sRGB, the external image display apparatus 13B displays the color space image data of adobe RGB and the external image display apparatus 13C displays the image data defined in other color space. When the color is adjusted by setting of the monitor 23 and when the image signal to be output to the monitor from the endoscope device 200 is directly input to each of the external image display apparatuses 13A, 13B and 13C, since color spaces of respective display images displayed on the external image display apparatuses 13A, 13B and 13C coincide with each other but settings of the monitor 23 are not able to be reflected in the external image display apparatus 13A and the color spaces of the respective display images are different from each other and also settings of the monitor 23 are not able to be reflected in the external image display apparatuses 13B and 13C, so that the color of the images displayed on the external image display apparatuses 13A, 13B and 13C become different from that of the display image displayed in the monitor 23 of the endoscope device 200.

Accordingly, the image signal to be output to the monitor is input to the capture device 300 having the display characteristic profile of the monitor 23. The capture device 300 obtains a corrected image signal obtained by correcting the image signal to be output to the monitor using the display characteristic profile and converts the corrected image signal into the standardization image based on the specific standard.

When the converted standardization image is input and when a standard of the standardization image is identical with that of the external image display apparatus, an image of the same color as that of the display image displayed on the monitor 23 of the endoscope device 200 is display in each of the external image display apparatuses 13A, 13 B and 13C. When the standard of the standardization image is different from that of the external image display apparatus, the image is displayed with a different color in the external image display apparatuses. In such a case, a color space conversion processing of the standardization image may be performed by the external image display apparatus to display the image having the same color.

Further, the standardization processing unit 37 (FIG. 1) may directly designate a specific standard to arbitrarily change the type of standard. That is, when the designated external image display apparatus is already known, the corrected image signal is converted into the standardization image signal based on the known standard. For example, when the sRGB is designated, the standardization processing unit 37 converts the corrected image signal based on the sRGB standard. Accordingly, the standardization image signal appropriate for the designated external image display apparatus is generated.

The image processing system 100 according to the present invention is not limited to a configuration in which the standardization image signal which is obtained by converting the image signal to be output to the monitor of the endoscope device 200 is output by the capture device 300. For example, the image processing system 100 may have a configuration in which the capture device 300 outputs the display characteristic profile and image signal to be output to the monitor to generate the standardization image signal by the external instrument connected to the network 15.

As described above, the present invention is not limited to embodiments described above and intends to include a combination of each configuration of the embodiments, modification or changes made by an ordinary skilled person in the art based on matters described in the specification or widely known technology and are included in matters for which protection is sought. The image signal to be output to the monitor output from the video processor 21 of the endoscope device 200 may be an image signal to be recorded in an external device output from an instrument other than the endoscope device 200. Further, a processing which generates the color correction image or the standardization image is not performed after acquiring the digital frame image, and may be a processing in which the color correction image or the standardization image is electrically corrected in a continuous analog signal stage.

As described above, the description of embodiments discloses the following matters.
(1) It is an image processing apparatus which receives from a modality, an image signal to be output to a displaying unit of the modality and outputs a standardization image signal obtained by standardizing the image signal to be output, the image processing apparatus including: a reference image signal outputting unit that outputs a reference image signal in which color information of a plurality of colors is included in a chart image in a display plane or a time axis; a color display information detection unit that detects color display information of an image displayed on the displaying unit based on the reference image signal; a profile generation unit that generates a display characteristic profile of the displaying unit using the detected color display information and the color information of the chart image; a display characteristic correcting unit that generates a color correction image signal obtained by correcting the image signal to be output which is input from the modality based on the display characteristic profile; and a standardization processing unit that converts the generated color correction image signal to the standardization image signal.
(2) It is the image processing apparatus of (1), in which the standardization processing unit that converts the generated color correction image signal to the standardization image signal of a specific color space.
(3) It is the image processing apparatus of (1) or (2), in which the modality is an endoscope device.
(4) It is an image processing system, including the image processing apparatus according to any one of (1) to (3); the modality provided with the displaying unit; and an external image display apparatus that displays the standardization image signal.
(5) It is the image processing system of (4), in which the standardization processing unit that converts the color correction image signal in a specific color space which is set as a reference by the external image display apparatus which becomes an output destination of the standardization image signal so as to generate a standardization image signal.
(6) It is an image processing method for receiving from a modality, an image signal to be output to a displaying unit of the modality and outputting a standardization image signal obtained by standardizing the image signal to be output, the image processing method including: outputting a reference image signal in which color information of a plurality of colors is included in a chart image in a display plane or a time axis; detecting color display information of an image displayed on the displaying unit based on the reference image signal; generating a display characteristic profile of the displaying unit using the detected color display information and the color information of the chart image; generating a color correction image signal obtained by correcting the image signal to be output which is input from the modality based on the display characteristic profile; and converting the generated color correction image signal to the standardization image signal.

## Claims

1. An image processing apparatus which receives from a modality, an image signal to be output to a displaying unit of the modality and outputs a standardization image signal obtained by standardizing the image signal to be output, the image processing apparatus comprising:
a reference image signal outputting unit that outputs a reference image signal in which color information of a plurality of colors is included in a chart image in a display plane or a time axis;
a color display information detection unit that detects color display information of an image displayed on the displaying unit based on the reference image signal;
a profile generation unit that generates a display characteristic profile of the displaying unit using the detected color display information and the color information of the chart image;
a display characteristic correcting unit that generates a color correction image signal obtained by correcting the image signal to be output which is input from the modality based on the display characteristic profile; and
a standardization processing unit that converts the generated color correction image signal to the standardization image signal.

2. The image processing apparatus of claim 1, wherein the standardization processing unit that converts the generated color correction image signal to the standardization image signal of a specific color space.

3. The image processing apparatus of claim 1 or 2, wherein the modality is an endoscope device.

4. An image processing system, comprising
the image processing apparatus according to any one of claims 1 to 3;
the modality provided with the displaying unit; and
an external image display apparatus that displays the standardization image signal.

5. The image processing system of claim 4, wherein the standardization processing unit that converts the color correction image signal in a specific color space which is set as a reference by the external image display apparatus which becomes an output destination of the standardization image signal so as to generate a standardization image signal.

6. An image processing method for receiving from a modality, an image signal to be output to a displaying unit of the modality and outputting a standardization image signal obtained by standardizing the image signal to be output, the image processing method comprising:
outputting a reference image signal in which color information of a plurality of colors is included in a chart image in a display plane or a time axis;
detecting color display information of an image displayed on the displaying unit based on the reference image signal;
generating a display characteristic profile of the displaying unit using the detected color display information and the color information of the chart image;
generating a color correction image signal obtained by correcting the image signal to be output which is input from the modality based on the display characteristic profile; and
converting the generated color correction image signal to the standardization image signal.
